# EUROPEAN PATENT APPLICATION

(11) **EP 4 014 957 A1**
(43) Date of publication of application: **22.06.2022**
(21) Application number: 20306632.9
(22) Date of filing: 21.12.2020
(51) Int. Cl.: A61K 8/44, A61Q 19/10, A61K 8/36

(54) **COMPOSITIONS COMPRISING COCAMIDOPROPYL BETAINE AND AT LEAST ONE FATTY ACID**

(71) Applicant: Johnson & Johnson Consumer Inc., Skillman, NJ 08558 (US)
(72) Inventor: GESLIN, Marie Cécile, 92130 Issy-les-Moulineaux (FR); PERRET, Cécile, 92130 Issy-les-Moulineaux (FR)
(74) Representative: Carpmaels & Ransford LLP

(57) **Abstract**

Provided are cleansing compositions comprising: (a) cocamidopropyl betaine, wherein the cocamidopropyl betaine has an average molecular weight of at least about 355 g/mol; (b) from greater than about 0 wt.-% to about 1 wt.-% of a fatty acid; and (c) water. Also provided are methods of using the same.

## Description

### FIELD

The present invention relates to compositions suitable for use in cleansing compositions. More specifically, the present invention relates to compositions comprising cocamidopropyl betaine and at least one fatty acid.

### BACKGROUND

Sodium lauryl ether sulfate, commonly referred to as "SLES," is an anionic surfactant with high foaming properties which is widely used in cosmetic products such as body washes and shampoos. It is a synthetic, non-biodegradable compound. As with many anionic surfactants, SLES may be an irritant for some users. Manufacturers have been looking for alternatives to SLES and other synthetic anionic surfactants to produce more sustainable products. One alternative is cocamidopropyl betaine, commonly referred to as "CAPB".

However, the foaming properties of CAPB are significantly lower when compared to anionic surfactants such as SLES. This is undesirable because the amount of foam generated, and the speed of foam generation, are important characteristics for the consumer. Thus, manufacturers have combined CAPB with an anionic surfactant such as SLES. These CAPB and SLES combinations result in products which are more sustainable than SLES alone, while preserving the consumer appealing foaming properties. However, increasing environmental concerns incite manufacturers to remove completely synthetic surfactant from their formulations while at the same time decreasing the amount of surfactants to reduce the environmental footprint and the costs of these formulations.

Therefore there is a need to provide a cleansing product, such as a cleansing composition, containing surfactants which are sustainable and non-irritating, while still having good cleansing and foaming properties.

### SUMMARY OF THE INVENTION

Accordingly, one aspect of the invention pertains to a cleansing composition comprising:
a. cocamidopropyl betaine, wherein the cocamidopropyl betaine has an average molecular weight of at least about 355 g/mol;
b. from greater than about 0 wt.-% to about 1 wt.-% of a fatty acid; and
c. water.

In one or more embodiments, the composition is essentially free of sodium lauryl ether sulfate. In some embodiments, the composition is essentially free of an anionic surfactant. In one or more embodiments, the cocamidopropyl betaine has an average molecular weight of no more than about 358 g/mol, preferably less than about 358 g/mol. In some embodiments, the cocamidopropyl betaine is present in an amount of about 1 wt.-% to about 5 wt.-%. In one or more embodiments, the composition further comprises a salt. In some embodiments, the salt is present in an amount of about 0.1 wt.-% to about 5 wt.-%. In one or more embodiments, the fatty acid is present in an amount of about 0.05 wt.-% to about 1 wt.-%. In some embodiments, the fatty acid has a C₁₂-C₁₆ carbon chain length. In one or more embodiments, the fatty acid is selected from the group consisting of lauric acid, palmitic acid, and combinations thereof. In some embodiments, the composition further comprises a non-ionic surfactant. In one or more embodiments, the non-ionic surfactant comprises PEG-80, sorbitan laurate, decyl glucoside and combinations thereof.

Another aspect of the invention pertains to a method of cleansing skin, the method comprising applying to the skin any of the cleansing compositions described herein. In some embodiments, the method further comprises lathering the cleansing composition to generate foam. In one or more embodiments, the method further comprises rinsing off the skin with water to wash off the cleansing composition and generated foam. In some embodiments, the skin is the skin of a human baby. In some embodiments, the method comprises applying to the skin a cleansing composition comprising:
a. cocamidopropyl betaine, wherein the cocamidopropyl betaine has an average molecular weight of from about 355 g/mol to about 357 g/mol;
b. from about 0.05 wt.-% to about 1 wt.-% of a fatty acid; and
c. water.

Another aspect of the invention pertains to a cleansing composition comprising:
a. from about 1 wt.-% to about 5 wt.-% cocamidopropyl betaine, wherein the cocamidopropyl betaine has an average molecular weight of from about 355 g/mol to about 357 g/mol;
b. from about 0.05 wt.-% to about 1 wt.-% of a fatty acid; and
c. water.

In some embodiments, the water is present in an amount of about 80 to about 90 wt.-%. In one or more embodiments, the composition further comprises one or more auxiliary agents selected from the group consisting of rheology modifiers, emulsifiers, preservatives, fragrances and combinations thereof.

These and other features and advantages of the present invention will be readily apparent from the following detailed description of the invention.

### DETAILED DESCRIPTION OF THE INVENTION

As used herein, "cosmetically / dermatologically acceptable" means suitable for use in contact with tissues (e.g., the skin or hair) without undue toxicity, incompatibility, instability, irritation, and/or allergic response, and the like.

As used herein, the term "safe and effective amount" means an amount sufficient to induce the desired effect, but low enough to avoid serious side effects. The safe and effective amount of the compound, extract, or composition will vary with, *e.g.,* the age, health and environmental exposure of the end user, the duration and nature of the treatment, the specific extract, ingredient, or composition employed, the particular pharmaceutically-acceptable carrier utilized, and like factors.

As used herein, "essentially free" or "substantially free" of an ingredient means containing less than 0.1 weight percent, or less than 0.01 weight percent, or none of an ingredient.

To provide a more concise description, some of the quantitative expressions given herein are not qualified with the term "about". It is understood that whether the term "about" is used explicitly or not, every quantity given herein is meant to refer to the actual given value, and it is also meant to refer to the approximation to such given value that would reasonably be inferred based on the ordinary skill in the art, including approximations due to the experimental and/or measurement conditions for such given value.

To provide a more concise description, some of the quantitative expressions herein are recited as a range from about amount X to about amount Y. It is understood that wherein a range is recited, the range is not limited to the recited upper and lower bounds, but rather includes the full range from about amount X through about amount Y, or any amount or range therein.

Accordingly, one aspect of the invention pertains to a cleansing composition comprising:
a. cocamidopropyl betaine, wherein the cocamidopropyl betaine has an average molecular weight of at least about 355 g/mol, preferably greater than about 355 g/mol;
b. from greater than about 0 wt.-% to about 1 wt.-% of a fatty acid; and
c. water.
It has been surprisingly discovered that the presence of such fatty acids enhances the foaming qualities of cocamidopropyl betaine, without the need for anionic surfactants, particularly sodium lauryl ether sulfate.

Cocamidopropyl betaine (also referred to "CAPB") is an amphoteric surfactant. CAPB is typically derived from coconut oil or palm kernel oil (or a mixture of both). Coconut oil is a mixture of several fatty acids and fatty esters having a range of carbon chain lengths. The majority of fatty acids have a carbon chain length ranging from C₈-C₁₈. Accordingly, one cocamidopropyl betaine molecule can have a different carbon chain length (and thus molecular weight) from another cocamidopropyl betaine molecule, depending on the fatty acids and fatty esters of the starting materials.

As used herein, the term "average molecular weight" of CAPB (also referred to as "Mw") represents the average molecular weight of the cocamidopropyl betaine present in a given composition. The CAPB Mw according to one or more embodiments of the invention is at least 355 g/mol, preferably greater than about 355 g/mol. The CACP Mw may be at least about 356 g/mol or at least about 357 g/mol. The CACB Mw may be no more than about 358 g/mol or no more than about 357 g/mol. In one or more embodiments, the Mw ranges from about 355 or 356 g/mol to about 357 or 358 g/mol (e.g. from about 355 g/mol to about 358 g/mol, or from about 355 g/mol to about 357 g/mol, or from about 356 g/mol to about 358 g/mol, or from about 356 g/mol to about 357 g/mol).

Mw is calculated by first analyzing the CPAB formulation by HPLC-CAD (High Performance Liquid chromatography with charged aerosol detection) to determine the fatty acid chain profile of the fatty acids bonded to the amidopropylbetaine moiety according to the following parameters and protocol shown in Table 1:

**Table 1: Process for Determining Fatty Acid Chain Profile Bonded to Amidopropylbetaine**

| | | | |
|---|---|---|---|
| Equipment | - Analytical balance, | | |
| | - Apparatus: HPLC with gradient system and Corona detector | | |
| | - Column: Acclaim Surfactant Plus 2.1 x 150 mm, 3µm from Thermo Fisher Scientific or equivalent | | |
| | - 50 mL volumetric flasks: class A or equivalent, | | |
| | - 0.45 µm filters: Acrodisc CR Gelman or equivalent | | |
| | - 1.5 ml amber color auto-sampler HPLC vials | | |
| | - 2-part disposable syringes (commonly used volume: 5 ml): Henke Sass Wolf Norm-Ject or equivalent | | |
| | - Magnetic stirrers: to be used for standard and sample preparation | | |
| Reagents | - De-ionized water for HPLC | | |
| | - Acetic Acid glacial RPE ACS for analysis from CARLO ERBA or equivalent | | |
| | - Acetonitrile RS ACS quality for HPLC from CARLO ERBA or equivalent | | |
| | - Ammonium Acetate RPE ACS for analysis from CARLO ERBA or equivalent | | |
| | | | |
| | Raw material references according to the raw material entering in finished products: | | |
| | - Cocamidopropyl Betaine reference: Dehyton PK 45 from BASF, with a % active matter range of 36.7-40.2%. | | |
| Reference solution preparation | Extraction solvent: Acetate buffer 0,05M pH 4/ Acetonitrile (70/30 v/v) with Acetate buffer = Ammonium Acetate 0,05M in deionized water, adjusted with Acetic Acid glacial at pH 4 | | |
| | | | |
| | Reference solution | | |
| | - In a 50 ml volumetric flask weigh about 120 mg of Dehyton PK 45 EAME | | |
| | - Complete to 50 ml with extraction solvent. | | |
| | - Mix well with a magnetic stirrer for at least 30 min. | | |
| | - Using a disposable syringe, filtrate on a 0.45 µm filter into a 1.5 ml HPLC vial. Seal the vial cap. | | |
| Sample | Extraction solvent: | | |
| preparation | Acetate buffer 0,05M pH 4/ Acetonitrile (70/30 v/v) with Acetate buffer = Ammonium Acetate 0,05M in deionized water, adjusted with Acetic Acid glacial at pH 4 Sample solution: | | |
| | - In a 50 ml volumetric flask, weigh accurately about X g of the product to be tested (for the weight, refer to the % active matter specified on the corresponding certificate of analysis). | | |
| | - Complete to 50 ml with extraction solvent. | | |
| | - Mix well with a magnetic stirrer for at least 30 min. | | |
| | - Using a disposable syringe, filtrate on a 0.45 µm filter into a 1.5 ml HPLC vial. Seal the vial cap. | | |
| Operating conditions | The startup of the Autosampler system must be realized before injection: | | |
| | • Fill the rinsing bottle with degassed fresh methanol, | | |
| | • Perform rinsing programs: | | |
| | - Prime Syringe: 10 cycles (twice), | | |
| | - Wash Buffer Loop, | | |
| | - Wash needle externally (50 µE). | | |
| | - Mobile phase: | | |
| | | Solvent A: Ammonium Acetate buffer (Ammonium Acetate 0.05M in deionized water adjusted with Acetic Acid glacial at pH=4) Solvent B: Acetonitrile | |
| | - Column temperature: 20°C | | |
| | - Flow rate: 0.4 ml/min | | |
| | -Analysis time: 29 min (for one HPLC run) | | |
| | -Injection volume: 5 µl | | |
| | - Corona parameters: nebulizer temperature: 25°C | | |
| | - Gradient elution: Enable the inverse gradient (the gradient for the Left Pump is automatically set up). Define the following gradient for the Right Pump: | | |

| | Time (min) | %A | %B |
|---|---|---|---|
| | 0 | 70 | 30 |
| | 2 | 70 | 30 |
| | 20 | 30 | 70 |
| | 27 | 30 | 70 |
| | 29 | 70 | 30 |
| Sample analysis Procedure | - After the injection of the reference solution, inject the sample solutions. | | |
| | - Integrate each peak without the blank peaks. | | |
| | - Measure % area values of each integrated peak | | |
| | - Calculate Global System Precision of the Reference solution | | |
| Calculation and reporting | The percent area of whole the peaks identified by numbers in the chromatograms are calculated and used for reporting | | |

This provides the percentage of C8-0 (caprylic acid), C10-0 (capric acid), C12-0 (lauric acid), C14-0 (myristic acid), C16-0 (palmitic acid), C18-0 (stearic acid), C18-1 (oleic acid), C18-2 (linoleic acid). Each fatty acid % is then multiplied by its molecular weight (i.e., corresponds to the fatty acid moiety molecular weight added to the amidopropylbetaine moiety molecular weight shown in Table 2 below). All the values are summed up and divided by 100 to provide the Mw value of the CAPB formulation. The Mw value provided is the weight average molecular weight.

**Table 2: CAPB Molecular Weights Used for Calculating Average Molecular Weight**

| | Molecular Weight |
|---|---|
| C8-0 | 286.5 |
| C10-0 | 314.5 |
| C12-0 | 342.5 |
| C14-0 | 370.6 |
| C16-0 | 398.6 |
| C18-0 | 426.7 |
| C18-1 | 424.7 |
| C18-2 | 422.6 |

The CAPB may be present in an amount of at least 1 (e.g. 1.0) wt.-%, or at least 1.5 wt.-%, or at least 2 (e.g. 2.0) wt.-%, or at least 2.5 wt.-%, or at least 3 (e.g. 3.0) wt.-% by total weight of the composition. The CAPB may be present in an amount of no more than 5 (e.g. 5.0) wt.-%, or no more than 4.5 wt.-%, or no more than 4 (e.g. 4.0), or no more than 3.5 wt.-%, or no more than 3 (e.g. 3.0) wt.-% by total weight of the composition. Accordingly, in some embodiments, the CAPB may be present in an amount ranging from about 1 (e.g. 1.0), 1.5, 2 (e.g. 2.0), 2.5 or 3 (e.g. 3.0) wt.-% to about 3 (e.g. 3.0), 3.5, 4 (e.g. 4.0), 4.5 or 5 (e.g. 5.0) wt.-% by total weight of the composition. In one or more embodiments, the CAPB is present in an amount ranging from about 2 to about 4 wt.-% (e.g. in an amount ranging from about 2.0 to about 4.0 wt.-%) by total weight of the composition.

Suitable commercially available cocamidopropyl betaines include Amphosol^{®} HCA from Stepan and Chembetain^{™} CAD from Lubrizol.

One or more of the compositions described herein comprise fatty acids. As used herein, the term "fatty acid" refers to a carboxylic acid having a long aliphatic chain. The fatty acid is a free fatty acid (*i.e.,* one that is not bound to another molecule). Fatty acids in accordance with one or more embodiments of the invention are those having a carbon chain length of from 8, 9, 10, 11, 12, 13, 14, 15 or 16 to 12, 13, 14, 15, 16, 17 or 18. In one or more embodiments, the fatty acid is selected from the group consisting of caprylic acid, capric acid, lauric acid, myristic acid, palmitic acid, stearic acid, and combinations thereof. In some embodiments, the fatty acids have a carbon chain length from 12 to 16. In one or more embodiments, the fatty acid is selected from the group consisting of lauric acid, palmitic acid, and combinations thereof. In one or more embodiments, the fatty acid is selected from the group consisting of lauric acid.

The fatty acids may be present in an amount of at least, or greater than, about 0.05, 0.06, 0.07, 0.1, or 0.2 wt.-% of the total weight of the composition. The fatty acids may be present in an amount of no more than 1, 0.9, 0.8, 0.7, 0.6, 0.5, 0.4, 0.35, 0.34 or 0.33 wt.-% by total weight of the composition. Accordingly, the fatty acids may be present in an amount of at least, or greater than, about 0, 0.05, 0.06, 0.07, 0.1, or 0.2 wt.-% to about 0.33, 0.34, 0.35, 0.4, 0.5, 0.6, 0.7, 0.8, 0.9, or 1 wt.-% by total weight of the composition. In some embodiments, the fatty acids may be present in an amount ranging from about 0 wt.-% to about 1 wt.-% by total weight of the composition. In one or more embodiments, the fatty acids may be present in an amount ranging from about 0.05 wt.-% to about 0.5 wt.-% by total weight of the composition.

As mentioned above, the presence of the fatty acid is able to enhance the foaming properties of the CAPB. This is advantageous because cleansing compositions containing CAPB usually need to be supplemented with anionic surfactants such as sodium lauryl ether sulfate to achieve foaming levels desired by consumers. Accordingly, in some embodiments, the compositions are essentially free, or free of, sodium lauryl ether sulfate. In one or more embodiments, the compositions are essentially free, or free of, an anionic surfactant.

The compositions described herein may comprise a salt. In some embodiments, the salt comprises NaCl. The salt (e.g., NaCl) may be present in an amount of at least about 0.1, 0.2, 0.3, 0.4, 0.5, 0.6, 0.7, 0.8, 0.9, or 1.0 wt.-% by total weight of the composition. The salt (e.g., NaCl) may be present in an amount of no more than 5 (e.g. 5.0), 4.5, 4 (e.g. 4.0), 3.5, 3 (e.g. 3.0), 2.5, 2 (e.g. 2.0) or 1.5 wt.-% by total weight of the composition. Accordingly, the salt (e.g., NaCl) may be present in an amount of from about 0.1, 0.2, 0.3, 0.4, 0.5, 0.6, 0.7, 0.8, 0.9, or 1.0 wt.-% to about 1 (e.g. 1.0), 1.5, 2 (e.g. 2.0), 2.5, 3 (e.g. 3.0), 3.5, 4 (e.g. 4.0), 4.5 or 5 (e.g. 5.0) wt.-% by total weight of the composition. In some embodiments, the salt (e.g., NaCl) may be present in an amount ranging from about 0.5 wt.-% to about 2 wt.-% (e.g. from about 0.5 wt.-% to about 2.0 wt.-%) by total weight of the composition. In one or more embodiments, the salt (e.g., NaCl) may be present in an amount ranging from about 0.5 wt.-% to about 2 wt.-% by total weight of the composition.

The cleansing compositions described herein contain water as a cosmetically-acceptable carrier. As used herein, the term "cosmetically-acceptable carrier" means a carrier that is suitable for use in contact with the skin without undue toxicity, incompatibility, instability, irritation, allergic response, and the like. Water may be present in an amount ranging from about 50, 55, 60, 65, 70, 75, 80, 85, or 90 % to about 60, 65, 70, 75, 80, 85, 90 or 95% by weight of the total composition. In some embodiments, water is present in an amount ranging from about 80 to 90 % by weight of the total composition.

The compositions can be formulated as solutions and may contain additional cosmetically-acceptable carriers (e.g., in addition to water). Solutions typically include an aqueous or organic solvent (e.g., from about 50% to about 99.99%, or from about 90% to about 99%, of a cosmetically acceptable aqueous or organic solvent). Examples of suitable organic solvents include: polyglycerols, propylene glycol, polyethylene glycol (200, 600), polypropylene glycol (425, 2025), glycerol, 1,2,4-butanetriol, sorbitol esters, 1,2,6-hexanetriol, ethanol, and mixtures thereof.

The compositions of the present invention may comprise any of a variety of additional other auxiliary ingredients used conventionally in healthcare/personal care compositions (referred to generally as "personal care components"). These other personal care components nonexclusively include one or more, pearlescent or opacifying agents, thickening agents, emollients, secondary conditioners, humectants, chelating agents, actives, exfoliants, and additives which enhance the appearance, feel and fragrance of the compositions, such as colorants, fragrances, preservatives, pH adjusting agents, rheology modifiers, and the like. Such auxiliary ingredients, when present, will be cosmetically / dermatologically acceptable and present in safe and effective amounts.

In some embodiments, the cleansing composition is essentially free or free of any surfactants other than the CAPB. What is meant by a surfactant is a surface-active agent intended to cleanse or emulsify. Examples of suitable surfactants include those found in Chapter 37, pages 431-450 (Classification of surfactants, by L. Oldenhove de Guertechin) in Handbook of Cosmetic Science and Technology (edited by A. Barel, M. Paye and H. Maibach, Published in 2001 by Marcel Dekker, Inc., New York, NY) and include, but are not limited to anionic, amphoteric, nonionic and cationic surfactants. Further, as one of the advantageous of the present invention is for the ability of CAPB to achieve superior foaming properties without the need of an additional anionic surfactant in particular, in some embodiments, the cleansing compositions are essentially free or free of an anionic surfactant. As used herein, the term "anionic surfactant" refers to a surfactant having a negatively charged hydrophilic polar group. Examples of anionic surfactants include ammonium lauryl sulfate, sodium laureth sulfate, sodium lauryl sarcosinate, sodium myreth sulfate, sodium pareth sulfate, sodium stearate, sodium lauryl sulfate, α olefin sulfonate, and ammonium laureth sulfate. In further embodiments, the cleansing compositions are essentially free or free of sodium lauryl ether sulfate.

Nevertheless, in some embodiments, the cleansing compositions may comprise one or more co-surfactants. As used herein, the term "co-surfactant" refers to a surfactant which is used together with the CAPB to support various aspects of the formulation, such as stability, structure and a desired level mildness. When present, the co-surfactant may be present in the cleansing composition in an amount of about 0.1, 0.2, 0.3, 0.4, 0.5, 0.6, 0.7, 0.8, 0.9, 1, 1.5, 2, 2.5 3, 3.5, 4, 4.5, or 5 to about 5, 5.5, 6, 6.5, 7, 7.5, 8, 8.5, 9, 9.5 or 10 weight percent of the composition.

Compositions of the present invention may comprise at least one nonionic surfactant as the co-surfactant. Examples of suitable nonionic surfactants include, but are not limited to the fatty alcohol acid or amide alkoxylates (e.g., propoxylates and/or ethoxylates), monoglyceride ethoxylates, sorbitan ester ethoxylates alkyl polyglycosides, and mixtures thereof. An example of a suitable amide alkoxylate type is PPG-2 hydroxyethyl cocamide, commercially available under the tradename Promidium^{™} CO from Croda. Another suitable nonionic surfactant is the polyoxyethylene derivatives of polyol esters, wherein the polyoxyethylene derivative of polyol ester (1) is derived from (a) a fatty acid containing from about 8 to about 22, and preferably from about 10 to about 14 carbon atoms, and (b) a polyol selected from sorbitol, sorbitan, glucose, α-methyl glucoside, polyglucose having an average of about 1 to about 3 glucose residues per molecule, glycerin, pentaerythritol and mixtures thereof, (2) contains an average of from about 10 to about 120, and preferably about 20 to about 80 oxyethylene units; and (3) has an average of about 1 to about 3 fatty acid residues per mole of polyoxyethylene derivative of polyol ester.

Examples of polyoxyethylene derivatives of polyol esters include, but are not limited to PEG-80, sorbitan laurate, and polysorbate 20. PEG-80 sorbitan laurate, which is a sorbitan monoester of lauric acid ethoxylated with an average of about 80 moles of ethylene oxide, is available commercially from ICI Surfactants of Wilmington, Del. under the tradename, "Atlas G-4280." Polysorbate 20, which is the laurate monoester of a mixture of sorbitol and sorbitol anhydrides condensed with approximately 20 moles of ethylene oxide, is available commercially from ICI Surfactants of Wilmington, Del. under the tradename "Tween 20."

Another class of suitable nonionic surfactants includes long chain alkyl glucosides or polyglucosides, which are the condensation products of (a) a long chain alcohol containing from about 6 to about 22, and preferably from about 8 to about 14 carbon atoms, with (b) glucose or a glucose-containing polymer. The alkyl gluocosides have about 1 to about 6 glucose residues per molecule of alkyl glucoside. A preferred glucoside is decyl glucoside, which is the condensation product of decyl alcohol with a glucose polymer and is available commercially from Henkel Corporation of Hoboken, N.J. under the tradename, "Plantaren 2000."

In some embodiments, the nonionic surfactant is selected from the group consisting of alkyl glucosides (e.g., decyl glucoside, lauryl glucoside, coco-glucoside) PEG-80, sorbitan Laurate and combinations thereof.

The compositions of the present invention may also contain at least one amphoteric surfactant (in addition to the CAPB) as the co-surfactant. As used herein, the term "amphoteric" shall mean: 1) molecules that contain both acidic and basic sites such as, for example, an amino acid containing both amino (basic) and acid (e.g., carboxylic acid, acidic) functional groups; or 2) zwitterionic molecules which possess both positive and negative charges within the same molecule. The charges of the latter may be either dependent on or independent of the pH of the composition. The amphoteric surfactants are disclosed herein without a counter ion. One skilled in the art would readily recognize that under the pH conditions of the compositions of the present invention, the amphoteric surfactants are either electrically neutral by virtue of having balancing positive and negative charges, or they have counter ions such as alkali metal, alkaline earth, or ammonium counter ions. Examples of amphoteric surfactants suitable for use in the present invention include, but are not limited to, amphocarboxylates such as alkylamphoacetates (mono or di); alkyl betaines; alkylamidoalkyl betaines (other than CAPB); alkylamidoalkyl sultaines; alkylamphophosphates; phosphorylated imidazolines such as phosphobetaines and pyrophosphobetaines; carboxyalkyl alkyl polyamines; alkylimino-dipropionates; alkylamphoglycinates (mono or di); alkylamphoproprionates (mono or di),); N-alkyl β-aminoproprionic acids; alkylpolyamino carboxylates; and mixtures thereof.

The compositions of the present invention may comprise at least one cationic surfactant as the co-surfactant. Classes of cationic surfactants that are suitable for use in this invention include alkyl quaternaries (mono, di, or tri), benzyl quaternaries, ester quaternaries, ethoxylated quaternaries, alkyl amines, and mixtures thereof, wherein the alkyl group has from about 6 carbon atoms to about 30 carbon atoms, with about 8 to about 22 carbon atoms being preferred. These cationic surfactants can be employed in composition of the present invention in an amount, based upon the total weight of the composition, from about 0.01% to about 20%, or from about 0.05% to about 15% or from about 0.1% to about 10%.

Any of a variety of commercially available pearlescent or opacifying agents which are capable of suspending water insoluble additives such as silicones and/or which tend to indicate to consumers that the resultant product is a conditioning shampoo are suitable for use in this invention. If used, the pearlescent or opacifying agent may be present in an amount, based upon the total weight of the composition, of from about 0.1 percent to about 10 percent, e.g. from about 1.5 percent to about 7 percent or from about 2 percent to about 5 percent. Examples of suitable pearlescent or opacifying agents include, but are not limited to mono or diesters of (a) fatty acids having from about 16 to about 22 carbon atoms and (b) either ethylene or propylene glycol; mono or diesters of (a) fatty acids having from about 16 to about 22 carbon atoms and (b) a polyalkylene glycol of the formula: HO-(JO)a-H, wherein J is an alkylene group having from about 2 to about 3 carbon atoms; and a is 2 or 3; fatty alcohols containing from about 16 to about 22 carbon atoms; fatty esters of the formula: KCOOCH₂L, wherein K and L independently contain from about 15 to about 21 carbon atoms; inorganic solids insoluble in the shampoo composition, and mixtures thereof.

The cleansing compositions described may also include any of a variety of conventional thickeners. Examples of suitable conventional thickeners include various thickeners having molecular weights of greater than about 100,000 grams per mole, including chemistries such as: hydroxyalkyl cellulose; alkyl cellulose; hydroxyalkyl alkyl cellulose; xanthan and guar gums, succinoglycan gums; Acrylates/C10-30 Alkyl Acrylate Crosspolymer; and mixtures thereof.

Examples of suitable thickening agents nonexclusively include: mono or diesters of 1) polyethylene glycol of formula: HO-(CH₂CH₂O)_{z}H, wherein z is an integer from about 3 to about 200; and 2) fatty acids containing from about 16 to about 22 carbon atoms; fatty acid esters of ethoxylated polyols; ethoxylated derivatives of mono and diesters of fatty acids and glycerin; hydroxyalkyl cellulose; alkyl cellulose; hydroxyalkyl alkyl cellulose; hydrophobically-modified alkali swellable emulsions (HASEs); hydrophobically-modified ethoxylated urethanes (HEURs); xanthan and guar gums; and mixtures thereof. Thickeners include polyethylene glycol ester, such as PEG-150 distearate which is available from the Hallstar Company of Chicago, Ill. under the tradename, "PEG 6000 DS".

Any of a variety of commercially available conditioners, such as volatile silicones, which impart additional attributes, such as gloss to the hair are suitable for use in this invention. The volatile silicone conditioning agent has an atmospheric pressure boiling point less than about 220 °C. The volatile silicone conditioner may be present in the invention, in ranges of from about 0 percent (if unused) to about 3 percent, e.g. from about 0.25 percent to about 2.5 percent or from about 0.5 percent to about 1.0 percent, based on the overall weight of the composition. Examples of suitable volatile silicones nonexclusively include polydimethylsiloxane, polydimethylcyclosiloxane, hexamethyldisiloxane, cyclomethicone fluids such as polydimethylcyclosiloxane available commercially from Dow Corning Corporation of Midland, Mich. under the tradename, "DC-345" and mixtures thereof, such as cyclomethicone fluids. Other suitable secondary conditioners include cationic polymers, including polyquarterniums, cationic guar, and the like. The present inventive compositions may be free of added silicones.

Any of a variety of commercially available humectants, which are capable of providing moisturization and conditioning properties to the personal cleansing composition, are suitable for use in the present invention. The humectant may be present in an amount of from about 0 percent (if unused) to about 10 percent, e.g. from about 0.5 percent to about 5 percent or from about 0.5 percent to about 3 percent, based on the overall weight of the composition. Examples of suitable humectants nonexclusively include: 1) water soluble liquid polyols selected from the group comprising glycerine, propylene glycol, hexylene glycol, butylene glycol, dipropylene glycol, polyglycerols, and mixtures thereof; 2) polyalkylene glycol of the formula: HO-(R" O)b-H, wherein R" is an alkylene group having from about 2 to about 3 carbon atoms and b is an integer of from about 2 to about 10; 3) polyethylene glycol ether of methyl glucose of formula CH₃-C₆H₁₀O₅-(OCH₂CH₂)_{c}-OH, wherein c is an integer from about 5 to about 25; 4) urea; and 5) mixtures thereof.

Examples of suitable chelating agents include those which are capable of protecting and preserving the compositions of this invention. The chelating agent may include ethylenediamine tetracetic acid ("EDTA"), such as tetrasodium EDTA, available commercially from Dow Chemical Company of Midland, Mich. under the tradename, "Versene 100XL". The chelating agent may be present in an amount, based upon the total weight of the composition, from about 0 (if unused) to about 0.5 percent or from about 0.05 percent to about 0.25 percent.

Suitable preservatives include, for example, parabens, quaternary ammonium species, phenoxyethanol, benzoates, DMDM hydantoin, and may be present in the composition in an amount, based upon the total weight of the composition, from about 0 (if unused) to about 1 percent or from about 0.05 percent to about 0.5 percent.

What is meant by an emollient is a compound that helps to maintain the soft, smooth, and pliable appearance of the skin (*e.g.,* by remaining on the skin surface or in the stratum corneum to act as a lubricant). Examples of suitable emollients include those found in Chapter 35, pages 399-415 (Skin Feel Agents, by G Zocchi) in Handbook of Cosmetic Science and Technology (edited by A. Barel, M. Paye and H. Maibach, Published in 2001 by Marcel Dekker, Inc New York, NY), and include, but are not limited to, petrolatum, hexyldecyl stearate, glyceryl oleate, and plant, nut, and vegetable oils such as macadamia nut oil, rice bran oil, grape seed oil, palm oil, prim rose oil, hydrogenates peanut oil, and avocado oil.

Any fragrance compositions suitable for use on skin may be used in the composition according to the present invention.

The cleansing composition may further optionally contain one or more benefit agents or pharmaceutically-acceptable salts thereof. As used herein, the term "benefit agent" includes any active ingredient that is to be delivered into and/or onto the skin, hair or nail at a desired location, such as a cosmetic agent or a therapeutic agent (such as a pharmaceutical agent or a medicament agent). By "cosmetic agent," it is meant any ingredient that is appropriate for cosmetically treating, providing nutrients to, and/or conditioning the hair, nail, and/or skin via topical application. The "cosmetic agent" may have a non-therapeutic effect. By "pharmaceutical agent," it is meant any drug that is either hydrophobic or hydrophilic in nature and appropriate for topical use. As used herein "medicament agents" include those agents capable of promoting recovery from injury and illness.

The benefit agents useful herein may be categorized by their therapeutic benefit or their postulated mode of action. However, it is to be understood that the benefit agents useful herein may, in some circumstances, provide more than one therapeutic benefit or operate via greater than one mode of action. Therefore, the particular classifications provided herein are made for the sake of convenience and are not intended to limit the benefit agents to the particular application(s) listed. In addition, the compounds, which are identified below as being suitable for use as benefit agents, may be used in an amount over and above the amount that they may be used for other purposes in the cleansing composition or personal care system.

Examples of suitable benefit agents include, but are not limited to, depigmentation agents; reflectants; film forming polymers; humectants; amino acids and their derivatives; antimicrobial agents; allergy inhibitors; anti-acne agents; anti-aging agents; anti-wrinkling agents, antiseptics; analgesics; antitussives; antipruritics; local anesthetics; anti-hair loss agents; hair growth promoting agents; hair growth inhibitor agents; antihistamines such as Mandragora Vernalis, Tanacetum Parthenium and the like; antiinfectives such as Acacia Catechu, Aloe Barbadensis, Convallaria Majalis, Echinacea, Eucalyptus, Mentha Piperita, Rosa Canina, Sassafras Albidum, and the like; inflammation inhibitors; anti-emetics; anticholinergics; vasoconstrictors; vasodilators; wound healing promoters; peptides, polypeptides and proteins; deodorants and anti-perspirants; medicament agents; skin emollients and skin moisturizers; skin firming agents, vitamins; tanning agents; skin lightening agents; antifungals such as Centaurea Cyanus, Kalmia Latifolia and antifungals for foot preparations; depilating agents; shaving preparations; external analgesics; perfumes; counterirritants; hemorrhoidals; insecticides; poison ivy products; poison oak products; burn products; anti-diaper rash agents; prickly heat agents; vitamins; herbal extracts; retinoids; flavenoids; sensates; anti-oxidants; skin conditioners; hair lighteners, chelating agents; cell turnover enhancers; coloring agents; sunscreens, those active ingredients disclosed in U.S. Pat. No. 6,063,397, which is incorporated herein by reference; anti-edema agents; collagen enhancers; and mixtures thereof.

Examples of suitable anti-edema agents nonexclusively include bisabolol natural, synthetic bisabolol, and mixtures thereof.

Examples of suitable vasoconstrictors nonexclusively include horse chestnut extract, prickly ash, and mixtures thereof.

Examples of suitable anti-inflammatory agents nonexclusively include benoxaprofen, centella asiatica, bisabolol, feverfew (whole), feverfew (parthenolide free), green tea extract, green tea concentrate, hydrogen peroxide, lycopene including "Lyc-o-Pen" available from LycoRed Natural Products Industries, Ltd., oat oil, chamomile, and mixtures thereof.

Examples of collagen enhancers nonexclusively include vitamin A, vitamin C, and mixtures thereof.

Examples of suitable skin firming agent nonexclusively include dimethylaminoethanol ("DMAE").

Examples of suitable antipruritics and skin protectants nonexclusively include oatmeal, betaglucan, feverfew, soy and derivatives thereof, bicarbonate of soda, colloidal oatmeal, surfactant based colloidal oatmeal cleanser, Anagallis Arvensis, Oenothera Biennis, Verbena Officinalis, and the like. These antipruritics may be used in an amount, based upon the total weight of the cleansing composition, from about 0.01 percent to about 40 percent, and preferably from about 1 percent to about 5 percent.

As used herein, colloidal oatmeal means the powder resulting from the grinding and further processing of whole oat grain meeting United States Standards for Number 1 or Number 2 oats. The colloidal oatmeal has a particle size distribution as follows: not more than 3 percent of the total particles exceed 150 micrometers in size and not more than 20 percent of the total particles exceed 75 micrometers in size. Examples of suitable colloidal oatmeals include, but are not limited to, "Tech-O" available from the Beacon Corporation and colloidal oatmeals available from Quaker.

Examples of suitable reflectants nonexclusively include mica, alumina, calcium silicate, glycol dioleate, glycol distearate, silica, sodium magnesium fluorosilicate, and mixtures thereof.

Suitable film forming polymers include acetyl tyrosinamide, zinc pyrithione, coal tar, benzoyl peroxide, selenium sulfide, hydrocortisone, sulfur, menthol, pramoxine hydrochloride, tricetylmonium chloride, polyquaternium 10, panthenol, panthenol triacetate, vitamin A and derivatives thereof, vitamin B and derivatives thereof, vitamin C and derivatives thereof, vitamin D and derivatives thereof, vitamin E and derivatives thereof, vitamin K and derivatives thereof, keratin, lysine, arginine, hydrolyzed wheat proteins, hydrolyzed silk proteins, octyl methoxycinnamate, oxybenzone, minoxidil, titanium dioxide, zinc dioxide, retinol, erthromycin, tretinoin, and mixtures thereof.

One type of benefit agent includes those therapeutic components that are effective in the treatment of dandruff, seborrheic dermatitis, and psoriasis as well as the symptoms associated therewith. Accordingly, the invention provides a cleansing composition for use in the therapeutic treatment of dandruff, seborrheic dermatitis, and psoriasis as well as the symptoms associated therewith, wherein the cleansing composition may comprise such a therapeutic component. Examples of such suitable benefits agents nonexclusively include zinc pyrithione, anthralin, shale oil and derivatives thereof such as sulfonated shale oil, selenium sulfide, sulfur; salicylic acid; coal tar; povidone-iodine, imidazoles such as ketoconazole, dichlorophenyl imidazolodioxalan, which is commercially available from Janssen Pharmaceutica, N.V., under the tradename, "Elubiol", clotrimazole, itraconazole, miconazole, climbazole, tioconazole, sulconazole, butoconazole, fluconazole, miconazole nitrate and any possible stereo isomers and derivatives thereof; piroctone olamine (Octopirox); selenium sulfide; ciclopirox olamine; anti-psoriasis agents such as vitamin D analogs, e.g. calcipotriol, calcitriol, and tacaleitrol; vitamin A analogs such as esters of vitamin A, e.g. vitamin A palmitate, retinoids, retinols, and retinoic acid; corticosteroids such as hydrocortisone, clobetasone, butyrate, clobetasol propionate and mixtures thereof.

### Methods

The present invention provides methods of treating and/or cleansing the human body comprising contacting at least a portion of the body with a composition of the present invention. For example, the present invention provides non-therapeutic methods of treating and/or cleansing the human body comprising contacting at least a portion of the body with a composition of the present invention. Also provided are the composition of the present invention for use in a therapeutic method of treating and/or cleansing the human body comprising contacting at least a portion of the body with the composition.

Certain methods comprising contacting mammalian skin and/or hair with a composition of the present invention to cleanse such region and/or treat such region for any of a variety of conditions including, but not limited to, acne, wrinkles, dermatitis, dryness, muscle pain, itch, and the like. Any of a variety of actives or benefit agents known in the art for treating such conditions may be used in the present invention. Given the gentleness of the CAPB as a cleansing surfactant, the compositions are particularly suited to cleansing the hair and skin of babies (*e.g*., children of less than 18, 15, 10, 5 4, 3, 2, or 1 years old).

For example, certain methods are non-therapeutic methods comprising contacting mammalian skin and/or hair with a composition of the present invention to cleanse such a region.

For example, certain methods are non-therapeutic methods comprising contacting mammalian skin and/or hair with a composition of the present invention to cleanse such a region and/or treat such a region for wrinkles and the like.

For example, the compositions of the present invention may be for use in a therapeutic method of cleansing and/or treating mammalian skin and/or hair for conditions including, but not limited to, acne, dermatitis, dryness, muscle pain, itch and the like, wherein the method comprises contacting such a region of mammalian skin and/or hair with the composition.

The cleansers described herein are useful in removing undesired elements from the skin of the user, such elements being anything to be removed from the skin, including, for example, dirt, oil, makeup, and potentially harmful bacteria. For example, after application of the cleanser onto the skin surface intended to be treated, the cleanser may be rubbed onto the target skin surface for about five revolutions with a finger or other cleaning device or tool, such as a pad, wipe, cloth, or other suitable device. In some aspects, the cleanser may be rubbed for about 10 revolutions.

In certain embodiments, the compositions produced via the present invention are preferably used as or in personal care products for treating or cleansing at least a portion of a human body. Examples of certain personal care products include various products suitable for application to the skin, hair, oral and/or perineal region of the body, such as shampoos, hand, face, and/or body washes, bath additives, gels, lotions, creams, and the like. Such products may further include a substrate onto which a composition is applied for use on the body. Examples of suitable substrates include a wipe, pouf, sponge, and the like as well as absorbent articles, such as a bandage, sanitary napkin, tampon, and the like.

The cleansing methods of the present invention may further comprise any of a variety of additional, optional steps associated conventionally with cleansing hair and skin including, for example, lathering, rinsing steps, and the like.

Thus, in some embodiments, the invention pertains to a method of cleansing skin, the method comprising applying to the skin one or more of the cleansing compositions described herein. The method may further comprise lathering the cleansing composition to generate foam. The method may also further comprise rinsing off the skin with water to wash off the cleansing composition and generated foam.

While the foregoing description represent exemplary embodiments of the present invention, it will be understood that various additions, modifications and substitutions may be made therein without departing from the spirit and scope of the present invention. In particular, it will be clear to those skilled in the art that the present invention may be embodied in other specific forms, structures, arrangements, proportions, and with other elements, materials, and components, without departing from the spirit or essential characteristics thereof. One skilled in the art will appreciate that the invention may be used with many modifications of structure, arrangement, proportions, materials, and components and otherwise, used in the practice of the invention, which are particularly adapted to specific environments and operative requirements without departing from the principles of the present invention. The presently disclosed embodiments are therefore to be considered in all respects as illustrative and not restrictive, the scope of the invention being indicated by the appended claims, and not limited to the foregoing description. It will be appreciated that in the claims, the term "comprises/comprising" does not exclude the presence of other elements or steps. In addition, singular references do not exclude a plurality. The terms "a", "an", "first", "second", etc., do not preclude a plurality.

All percentages, parts and ratios are based upon the total weight of the composition of the present invention, unless otherwise specified. All such weights as they pertain to the listed ingredients are based on the level of the particular ingredient described and, therefore, do not include carriers or by-products that may be included in commercially available materials, unless otherwise specified.

### EXAMPLES

The following test methods and materials were used in the Examples.

### EXAMPLE 1: Preparation of CAPB Formulations

Several CAPB formulations were prepared. This began with the preparation of several formulations containing CAPB, fatty acids, NaCl, glycerin and water. Several commercially available CAPB raw materials were used to create CAPB formulations A-J with the ingredients and in the amounts shown in Table 3 below. The fatty acid content was a blend, unless otherwise noted.

**Table 3: CAPB Formulations**

| **CAPB formulations** | **CAPB Active Matter** | **Fatty Acids** | **NaCl** | **Glycerin** | **Water** | **Average CAPB Molecular Weight (Mw)** |
|---|---|---|---|---|---|---|
| A | 35.9% | 1.9% | 4.7% | 2% | Q.S. | 351.5 |
| B | 35% | 1.8% | 7.1% | 2.2% | Q.S. | 353.5 |
| C | 35% | 2.2% | 4.7% | 0% | Q.S. | 356.5 |
| D | 31% | 0.35% | 7.1% | - | Q.S. | 351.2 |
| E | 30.2% | 1% (C16) | 5.9% | 0% | Q.S. | 356.6 |
| F | 30.2% | 1% (C12) | 5.9% | 0% | Q.S. | 356.6 |
| G | 30.1% | 0% | 7.1% | 0% | Q.S. | 356.6 |
| H | 34.1% | 2.16% | 7.14% | 0% | Q.S. | 356.5 |
| I | 33.1% | 0.9% | 5.4% | 1.1% | Q.S | 355.05 |
| J | 32.4% | 2.88% | 5.3% | 1.07% | Q.S | 355.05 |

Mw was calculated by analyzing the CPAB formulation by HPLC-CAD (High Performance Liquid chromatography with charged aerosol detection) according to the process described above. This provided the percentage of C8-0 (Caprylic acid), C10-0 (Capric acid), C12-0 (lauric acid), C14-0 (Myristic acid), C16-0 (Palmitic acid), C18-0 (Stearic acid), C18-1 (Oleic acid), C18-2 (Linoleic acid). Each fatty acid % was then multiplied by its molecular weight (*i.e.,* corresponds to the fatty acid moiety molecular weight added to the amidopropylbetaine moiety molecular weight), as shown in Table 4. All the values are summed up and divided by 100 to provide the Mw value of the CAPB formulation.

**Table 4: CAPB Molecular Weights Used for Calculating Average Molecular Weight**

| | Molecular Weight |
|---|---|
| C8-0 | 286.5 |
| C10-0 | 314.5 |
| C12-0 | 342.5 |
| C14-0 | 370.6 |
| C16-0 | 398.6 |
| C18-0 | 426.7 |
| C18-1 | 424.7 |
| C18-2 | 422.6 |

### EXAMPLE 2: Preparation of Cleansing Compositions

The CAPB formulations from Example 1 were used to prepare several cleansing compositions. Three different cleansing composition chassis were used to create cleansing compositions C1-4 and 11-8, having the ingredients and Mw values shown in Tables 5-7 below. C1-4 are comparative examples. C1, C3 and C4 are considered to be comparative because of the Mw value. C2 is considered to be comparative because it does not contain any fatty acid.

**Table 5: Chassis 1 Cleansing Compositions**

| | C1 | C2 | C3 | C4 | I1 | 12 | 13 | 14 | 15 | 16 |
|---|---|---|---|---|---|---|---|---|---|---|
| CAPB Formulation | A | G | D | B | C | I | H | J | E | F |
| | | | | | | | | | | |

| INCI Name | | | | | | | | | | |
|---|---|---|---|---|---|---|---|---|---|---|
| Water | 85.93 | 86.79 | 86.63 | 85.77 | 86.25 | 86.39 | 86.08 | 86.26 | 86.79 | 86.79 |
| Glycerin | 0.23 | 0.00 | 0.00 | 0.25 | 0.00 | 0.13 | 0.00 | 0.12 | 0.00 | 0.00 |
| Sodium Chloride | 0.54 | 0.80 | 0.81 | 0.81 | 0.53 | 0.62 | 0.80 | 0.61 | 0.67 | 0.67 |
| Cocami dopropyl Betaine | 4.13 | 3.46 | 3.57 | 4.02 | 4.02 | 3.81 | 3.92 | 3.73 | 3.47 | 3.47 |
| Coconut Acids¹ | 0.22 | 0.00 | 0.04 | 0.20 | 0.08 | 0.10 | 0.08 | 0.10 | 0.00 | 0.00 |
| Lauric Acid (C12:0) | 0.00 | 0.00 | 0.00 | 0.00 | 0.17 | 0.00 | 0.17 | 0.23 | 0.00 | 0.12 |
| Palmitic acid (C16:0) | 0.00 | 0.00 | 0.00 | 0.00 | 0.00 | 0.00 | 0.00 | 0.00 | 0.12 | 0.00 |
| Co-Surfactants (PEG-80 Sorbitan Laurate, Decyl Glucoside, Coco-glucoside) | 5.82 | 5.82 | 5.82 | 5.82 | 5.82 | 5.82 | 5.82 | 5.82 | 5.82 | 5.82 |
| Acrylates/C10-30 Alkyl Acrylate Crosspolymer | 0.80 | 0.80 | 0.80 | 0.80 | 0.80 | 0.80 | 0.80 | 0.80 | 0.80 | 0.80 |
| Glyceryl Oleate | 0.30 | 0.30 | 0.30 | 0.30 | 0.30 | 0.30 | 0.30 | 0.30 | 0.30 | 0.30 |
| Gossypium Herbaceum (Cotton) | 0.25 | 0.25 | 0.25 | 0.25 | 0.25 | 0.25 | 0.25 | 0.25 | 0.25 | 0.25 |
| Misc. (fragrance, preservative, buffers) | 1.78 | 1.78 | 1.78 | 1.78 | 1.78 | 1.78 | 1.78 | 1.78 | 1.78 | 1.78 |
| | | | | | | | | | | |
| Total | 100.00 | 100.00 | 100.00 | 100.00 | 100.00 | 100.00 | 100.00 | 100.00 | 100.00 | 100.00 |
| Mw | 351.5 | 356.6 | 351.2 | 353.5 | 356.5 | 355.05 | 356.5 | 355.05 | 356.6 | 356.6 |

| | | | | | | | | | | |
|---|---|---|---|---|---|---|---|---|---|---|
| 1. Coconut acids are a mixture of fatty acids found in coconut oil | | | | | | | | | | |

**Table 6: Chassis 2 Cleansing Composition**

| | 17 |
|---|---|
| CAPB Formula | F |
| | |
| INCI Name | |
| Aqua | 91.75 |
| Glycerin | 2 |
| Sodium Chloride | 2 |
| Cocamidopropyl Betaine | 2.03 |
| Lauric Acid (C12:0) | 0.07 |
| Polysorbate 20 | 0.2 |
| Polyquaternium-7 | 0.09 |
| Misc. (fragrance, preservative, buffers, etc.) | 1.86 |
| Total | 100.00 |
| Mw | 356.6 |

**Table 7: Chassis 3 Cleansing Composition**

| | 18 |
|---|---|
| CAPB Formula | F |
| | |
| INCI Name | |
| Aqua | 87.08 |
| Sodium Chloride | 0.76 |
| Cocamidopropyl Betaine | 3.83 |
| Lauric Acid (C12:0) | 0.11 |
| PEG-150 Pentaerythrityl Tetrastearate | 1.35 |
| PPG-2 Hydroxyethyl Cocamide | 0.75 |
| Co-Surfactants (PEG-80 Sorbitan Laurate, Decyl Glucoside) | 3.92 |
| Sodium Methyl Cocoyl Taurate | 0.52 |
| Misc. (fragrance, preservative, buffers, etc.) | 1.68 |
| Total | 100.00 |
| Mw | 356.6 |

### EXAMPLE 3: Foam Test

The cleansing compositions of Example 2 were then subjected to a foam test to determine foaming ability. The foam test was a cylinder shake foam test. Details of the testing protocol of the test follows below.

### Foam measure: Cylinder Shake Foam Test

The apparatus for the Cylinder Shake Foam Test was custom built by Gaum Inc. The apparatus holds up to four 500-mL graduated cylinders which are stoppered and clamped in place on a plate which can be oscillated repeatedly from a vertical to an inverted position. Oscillation speed can be set between 4 and 20 cycles/min.

### Test Variables:

- test sample size: 2.50 gm
- DI water (room temperature) plus sample filled to 250 mL mark on cylinder
- Total cycles set to stop at 10
- oscillation speed was approximately 13 cycles per minute using "Speed" setting of 32.5

### Test Parameters:

- Flash Foam: net foam assessed after 1 cycle
- Total Foam (liquid + foam): assessed at end of 10th cycle
- Total Foam (foam only): net foam assessed after 10th cycle was also determined
- Foam Decay: net foam loss after 5 minutes from end of 10th cycle
- 3 replicates were tested for each sample
- Digital images were taken at each assessment point for consistent determination of foam volumes which are subjective.

### Procedure:

1) place approximately 240 gm of DI water into one of the test cylinders
2) add 2.50 gm of test sample to the water in the test cylinder
3) stopper the cylinder and clamp onto the desired location in the test apparatus
4) repeat for each cylinder to be tested simultaneously and close safety doors
5) set instrument controls for 10 cycles and "Auto"
6) "Start" test but "Stop" after 1 cycle
7) open safety doors and capture image of cylinders
8) close safety doors and press "Start" to resume cycles which will stop automatically at the end of the 10th cycle
9) start timer for 5 minutes
10) open safety doors and capture image of cylinders
11) when 5 minutes delay ends, capture image of cylinders
12) clean cylinder and set up next set of samples

The results of the foam test are shown below in Tables 8-9 below.

**Table 8: Comparative Examples Foam Test Results**

| | C1 | C2 | C3 | C4 |
|---|---|---|---|---|
| CAPB formulation | A | G | D | B |
| Chassis | 1 | 1 | 1 | 1 |
| | | | | |
| Cocami dopropyl Betaine | 4.13% | 3.46% | 3.57% | 4.02% |
| NaCl | 0.54% | 0.80% | 0.81% | 0.81% |
| Fatty acid | 0.22% | 0.00% | 0.04% | 0.20% |
| Mw | 351.5 | 356.6 | 351.2 | 353.5 |
| Flash Foam [mL] | 40 | 35 | 40 | 35 |
| Total Foam [mL] | 475 | 375 | 375 | 375 |

**Table 9: Inventive Examples Foam Test Results**

| | I1 | I2 | I3 | I4 | I5 | I6 | I7 | I8 |
|---|---|---|---|---|---|---|---|---|
| CAPB formulation | C | I | H | J | E | F | F | F |
| Chassis | 1 | 1 | 1 | 1 | 1 | 1 | 2 | 3 |
| | | | | | | | | |
| Cocamidopropyl Betaine | 4.02% | 3.81% | 3.92% | 3.73% | 3.47% | 3.47% | 2.03% | 3.83% |
| NaCl | 0.53% | 0.62% | 0.80% | 0.61% | 0.67% | 0.67% | 2% | 0.76% |
| Fatty acid | 0.25% | 0.10% | 0.25% | 0.33% | 0.12% | 0.12% | 0.07% | 0.11% |
| Mw | 356.5 | 355.05 | 356.5 | 355.05 | 356.6 | 356.6 | 356.6 | 356.6 |
| Flash Foam [mL] | 65 | 65 | 60 | 68.5 | 61.1 | 69.6 | 69.0 | 70.8 |
| Total Foam [mL] | 450 | 375 | 350 | | | | | |

As can be seen from the above, the inventive examples exhibited a much higher flash foam (ranging from 60-70.8 mL) compared to the comparative examples (which ranged from 35-40 mL). A higher flash foam indicates the ability of the formulation to foam. As described above, this is advantageous because CAPB is gentler on skin than traditional surfactants but is also known not to foam as much. Thus, an increase of foaming ability of a cleansing composition is very desirable. Good foaming properties were seen in all three chassis.

Further, the data show that the CAPB average molecular weight affects the flash foam results. The flash foam was improved by more than 50% in changing the CAPB average molecular weight where all other parameters were kept either the same or similar. This was shown particularly by comparing the results from C1 and C4 to I1 and I3, respectively. A summary of these examples is reproduced below in Table 10. This is a surprising result because one of ordinary skill in the art would think that longer carbonated chain decreases the amount of foam generated, and thus would impact the flash foam negatively.

**Table 10: C1 vs. I1 and C4 vs. I3**

| | C1 | I1 | C4 | 13 |
|---|---|---|---|---|
| CAPB formulation | A | C | B | H |
| Chassis | 1 | 1 | 1 | 1 |
| | | | | |
| NaCl | 0.54% | 0.53% | 0.81% | 0.80% |
| Fatty acid | 0.22% | 0.25% | 0.20% | 0.25% |
| Mw | 351.5 | 356.5 | 353.5 | 356.5 |
| Flash Foam mL | 40 | 65 | 35 | 60 |
| Total Foam mL | 475 | 450 | 375 | 350 |

The data also show that the presence of fatty acid boosts the flash foam. This was shown particularly by comparing the results from C2 and I3. A summary of these examples is reproduced below in Table 11. This is a surprising result because one of ordinary skill in the art would think that the presence of fatty acid decreases the amount of foam generated, and thus would impact the flash foam negatively.

**Table 11: C2 vs. I3**

| | C2 | I3 |
|---|---|---|
| CAPB formulation | G | H |
| Chassis | 1 | 1 |
| | | |
| NaCl | 0.80% | 0.80% |
| Fatty acid | 0% | 0.25% |
| Mw | 356.6 | 356.5 |
| Flash Foam mL | 35 | 60 |
| Total Foam mL | 375 | 350 |

Additionally, a comparison of I5 and I6 demonstrates the effect of changing length of fatty acid. A summary of these examples is reproduced below in Table 12. I5 and I6 are exactly the same except that the fatty acid of I5 is entirely palmitic acid (C16) and that of I6 is entirely lauric acid (C12). There is further improvement in flash foam when lauric acid is used instead of palmitic acid.

**Table 12: I5 vs. I6**

| | I5 | I6 |
|---|---|---|
| CAPB formulation | E | F |
| Chassis | 1 | 1 |
| | | |
| Cocamidopropyl Betaine | 3.47% | 3.47% |
| NaCl | 0.67% | 0.67% |
| Fatty acid | 0.12% (Palmitic acid) | 0.12% (Lauric acid) |
| Mw | 356.6 | 356.6 |
| Flash Foam [mL] | 61.1 | 69.6 |

## Claims

1. A cleansing composition comprising:
a. cocamidopropyl betaine, wherein the cocamidopropyl betaine has an average molecular weight of at least about 355 g/mol;
b. from greater than about 0 wt.-% to about 1 wt.-% of a fatty acid; and
c. water.

2. The cleansing composition of claim 1 or claim 2, wherein the composition is essentially free of sodium lauryl ether sulfate,
preferably wherein the composition is essentially free of an anionic surfactant.

3. The cleansing composition of any preceding claim, wherein the cocamidopropyl betaine has an average molecular weight of no more than 358 g/mol,
preferably less than about 358 g/mol.

4. The cleansing composition of any preceding claim, wherein the cocamidopropyl betaine is present in an amount of about 1 wt.-% to about 5 wt.-%.

5. The cleansing composition of any preceding claim further comprising a salt, preferably wherein the salt is present in an amount of about 0.1 wt.-% to about 5 wt.-%.

6. The cleansing composition of any preceding claim, wherein the fatty acid is present in an amount of about 0.05 wt.-% to about 1 wt.-%.

7. The cleansing composition of any preceding claim, wherein the fatty acid has a C₁₂-₁₆ carbon chain length,
preferably wherein the fatty acid is selected from the group consisting of lauric acid, palmitic acid, and combinations thereof.

8. The cleansing composition of any preceding claim, further comprising a non-ionic surfactant,
preferably wherein the non-ionic surfactant comprises PEG-80, sorbitan laurate, decyl glucoside or combinations thereof.

9. A cleansing composition comprising:
a. from about 1 wt.-% to about 5 wt.-% cocamidopropyl betaine, wherein the cocamidopropyl betaine has an average molecular weight of about 355 g/mol to about 357 g/mol;
b. from about 0.05 wt.-% to about 1 wt.-% of a fatty acid; and
c. water.

10. The cleansing composition of any preceding claim, wherein the water is present in an amount of about 80 to about 90 wt.-%.

11. The cleansing composition of any preceding claim, further comprising one or more auxiliary agents selected from the group consisting of rheology modifiers, emulsifiers, preservatives, fragrances, and combinations thereof.

12. A method of cleansing skin, the method comprising applying to the skin the cleansing composition of any one of claims 1 to 11.

13. The method of claim 12, further comprising lathering the cleansing composition to generate foam,
preferably further comprising rinsing off the skin with water to wash off the cleansing composition and generated foam.

14. The method of any one of claims 12 to 13, wherein the skin is the skin of a human baby.

15. The method of any one of claims 12 to 14, wherein the cleansing composition comprises:
a. cocamidopropyl betaine, wherein the cocamidopropyl betaine has an average molecular weight of about 355 g/mol to about 357 g/mol;
b. from about 0.05 wt.-% to about 1 wt.-% of a fatty acid; and
c. water.
